# EUROPÄISCHE PATENTSCHRIFT

(11) **EP 1 307 168 B1**
(45) Veröffentlichungstag und Bekanntmachung des Hinweises auf die Patenterteilung: **06.04.2005**
(21) Anmeldenummer: 01971902.0
(22) Anmeldetag: 10.08.2001
(51) Int. Cl.: A61H 9/00, A61H 1/02, A63B 22/00, A63B 22/08, A61G 10/02, A63B 21/005

(54) **VORRICHTUNG ZUM SCHLANKMACHEN**
SLIMMING DEVICE
DISPOSITIF D'AMINCISSEMENT

(30) Priorität: 10.08.2000 AT 13832000; 28.03.2001 DE 10115286; 28.03.2001 US 818999
(43) Veröffentlichungstag der Anmeldung: 07.05.2003
(73) Patentinhaber: Egger, Norbert, Dr., 5020 Salzburg (AT)
(72) Erfinder: Egger, Norbert, Dr., 5020 Salzburg (AT)
(74) Vertreter: Grünecker, Kinkeldey, Stockmair & Schwanhäusser Anwaltssozietät
(86) Internationale Anmeldenummer: PCT/EP2001/009281
(87) Internationale Veröffentlichungsnummer: WO 2002/011661

(56) Entgegenhaltungen:
- EP-A- 0 906 774
- FR-A- 1 163 577
- FR-A- 2 618 342
- US-A- 1 336 774
- US-A- 5 133 339
- PATENT ABSTRACTS OF JAPAN vol. 1996, no. 09, 30. September 1996 (1996-09-30) & JP 08 112373 A (SATO KOGYO CO LTD;OOKURA SHOJI KK), 7. Mai 1996 (1996-05-07)

## Beschreibung

Die vorliegende Erfindung betrifft eine Vorrichtung zum Schlankmachen von Personen durch Verbesserung der Hautdurchblutung mittels Unterdruck ohne zusätzliche Gelenkbelastung. Die Vorrichtung umfasst hierbei ein Gehäuse, in dem eine mit den Beinen betätigbare Arbeitseinrichtung und ein Sitz zur Abstützung des Körpers der Person angeordnet ist, eine Öffnung in dem Gehäuse, welche den Körper der Person umschließt, eine Dichtmanschette zur Abdichtung des Gehäuses im Bereich der Öffnung gegenüber dem Körper der Person, und eine Vakuumpumpe zur Erzeugung eines Unterdrucks in dem Gehäuse. Darüber hinaus umfasst die Erfindung ein Verfahren zur Körperertüchtigung von Personen.

Eine derartige Vorrichtung ist zum Beispiel aus der EP 0 906 774 A bekannt. Hierbei können mit der bekannten Vorrichtung verschiedene Übungen durchgeführt und Therapiekonzepte verwirklicht werden. Der Unterdruck, der während der Betätigung des Gerätes auf die untere Körperhälfte der Person einwirkt, hat eine stimulierende Wirkung, wobei insbesondere die Durchblutung der Haut gefördert wird. Daher kann beispielsweise ein gezielter Abbau des Fettgewebes erreicht werden. Durch die Einstellung des Unterdrucks, gegebenenfalls auch eines zeitweise angewendeten Überdrucks, der Belastung der Person durch das Arbeitsgerät und durch die Klimatisierung der Kammer kann die Therapiewirkung an die jeweiligen Bedürfnisse angepasst werden. Die therapeutische Wirkung wird bei der Vorrichtung der EP 0 906 774 A in erster Linie durch den Unterdruck erreicht.

Obgleich die bekannte Vorrichtung gute Ergebnisse liefert und bei einer Vielzahl von Indikationen anwendbar ist, hat sich dennoch das Bedürfnis nach einer noch selektiveren Anwendungsmöglichkeit entwickelt.

Ferner ist aus der US 5,133,339 ein Trainingsgerät mit einem Laufband bekannt, das insbesondere für den Einsatz im schwerelosen Raum bestimmt ist. Da der Anpressdruck der Person auf das Laufband durch den im Gerät eingestellten Unterdruck bestimmt ist, sind insbesondere im Bereich niedriger spezifischer Belastung enge Grenzen gesetzt. Das Gerät kann hierbei sowohl für das Training als auch zur Rehabilitation eingesetzt werden, da hier eine Belastung des muskoskelettalen Gewebes möglich ist, so dass ein Wachstum der Muskel- und Knochenmasse gefördert wird. Entscheidend ist hierbei zur Erzielung der Wirkung, dass Trainingsgeräte verwendet werden, die im Stehen oder Gehen betrieben werden, da der Bewegungsapparat des Menschen durch Simulation erhöhter oder verringerter Schwerkraft trainiert werden soll. Ohne die Betätigung im Stehen ist eine Simulation der Schwerkraftwirkung nicht möglich.

Nachteilig bei der Vorrichtung der US 5,133,339 ein Trainingseffekt nur unter sehr starker Belastung der Gelenke möglich ist. Um eine Überlastung der Gelenke zu verhindern, muss bei der Vorrichtung der US 5,133,339 der Unterdruck in der Vorrichtung wieder aufgehoben werden.

Aufgabe der vorliegenden Erfindung ist es, die oben beschriebene Vorrichtung so weiterzubilden, dass eine verschlankende Wirkung auf die Benutzer ausgeübt wird, ohne die Gelenke übermäßig stark zu beanspruchen.

Ferner soll eine möglichst weitgehende Anpassung an speziell ausgearbeitete Therapiekonzepte oder Übungsprogramme ermöglicht werden.

Diese Aufgabe wird erfindungsgemäß durch die Merkmale des Anspruchs 1 gelöst.

Es hat sich herausgestellt, dass der Unterdruck in der Kammer sowohl eine lokale als auch eine systemische Wirkung auf die Person ausübt, welche die Arbeitseinrichtung in der Vorrichtung betreibt. Das Ausmaß der systemischen Wirkung ist jedoch im Bereich der Extremitäten unterschiedlich zu dem im Bereich des Körperstammes, und überdies sind die lokalen Verhältnisse, wie etwa der Aufbau des Fettgewebes, unterschiedlich. Durch die Einstellung unterschiedlicher Druckniveaus kann dieser Tatsache in optimaler Weise Rechnung getragen werden. Besonders günstig ist hierbei, das die Druckverhältnisse während der Dauer einer Übungseinheit einen unterschiedlichen zeitlichen Verlauf haben können, so das mehrere Parameter unabhängig voneinander optimiert werden können.

Ferner hat es sich als günstig erwiesen, wenn die erste Dichtmanschette zur Abdichtung im Bereich der Hüfte der Person ausgebildet ist, während die zweite Dichtmanschette zur Abdichtung im Bereich des Brustkorbs der Person ausgebildet ist. Die zweite Dichtmanschette wird dabei im allgemeinen bis in den Bereich unterhalb der Achseln der Person hochgezogen sein. Dadurch kann der Bauchbereich und ein großer Teil des Brustkorbs separat beeinflusst werden.

Für einen einfachen Transport und für die Aufstellung in Fitness-Studios, in denen oftmals beengte Raumverhältnisse herrschen, soll das Gerät in einer besonders vorteilhaften Ausgestaltung möglichst kompakt ausgestaltet sein. Diese Aufgabe wird erfindungsgemäß dadurch gelöst, dass das Gehäuse als Rundkörper, insbesondere eiförmig, ausgebildet ist.

Die Ausgestaltung des Gehäuses als Rundkörper stellt einerseits eine besonders kompakte Bauform zur Verfügung, gleichermaßen weist der Rundkörper die besten Eigenschaften bei Druckeinwirkung auf. Auf diese Weise kann auch bei relativ dünnen Wandstärken eine möglichst hohe Stabilität erreicht werden. Der Einsatz dünnerer Wandstärken wirkt sich gleichermaßen vorteilhaft auch auf die Herstellungskosten und das Gewicht der Vorrichtung aus.

Als besonders vorteilhaft hat es sich hierbei herausgestellt, dass das Gehäuse in Halbschalen teilbar ist, wobei jede Halbschale einen Teil der Öffnung aufweist, die durch ein Verschlusssystem miteinander verbunden werden. Durch die Teilbarkeit des Gerätes ist es möglich, diese besonders leicht und z.B. auch durch schmale Türöffnungen zu transportieren. Hierbei ermöglicht ein Schnellverschlusssystem ein rasches Zerlegen und Zusammenstellen des Gerätes.

Gleichermaßen ermöglicht die Ausgestaltung des Gehäuses in zwei Hälften, die jeweils einen Teil der Öffnung aufweisen, ein leichtes Einsteigen in die Vorrichtung, da die beiden Teile z. B. zum Einsteigen auseinandergeschoben werden können.

Ferner hat es sich als vorteilhaft erwiesen, wenn an der Öffnung ein Bereich des Gehäuses als Türelement ausgebildet ist. Auch auf diese Weise wird ein sehr leichtes Ein- und Aussteigen aus dem Gerät gewährleistet. Dabei ist es denkbar, das Türelement als nach unten aufklappendes Element auszubilden und auf der aufgeklappten Seite Treppenelemente anzuordnen, um das Einsteigen noch weiter zu erleichtern.

Gemäß einer anderen bevorzugten Ausführungsform ist die Öffnung seitlich angeordnet und es erstreckt sich durch die Öffnung in das Gehäuse eine Liegeeinrichtung, wobei die Person während der körperlichen Ertüchtigung eine liegende Position einnimmt. Durch die liegende Position wird das Gesäß vom Körpergewicht entlastet und ermöglicht so ein Trainieren weitgehend ohne Anpressdruck. Diese Ausführungsform ist unabhängig von den obigen Ausgestaltungen vorteilhaft:

Gerade im Zusammenwirken mit dem Unterdruck, der verstärkt Blut in den mit Unterdruck beaufschlagten Bereich des Körpers saugt, bietet die liegende Position besondere Vorteile. In der liegenden Position sind die Beine der Person nicht unterhalb des Rumpfes angeordnet, so dass ein Rückfluss des Bluts zum Herzen hin gefördert wird. Die Beinposition führt also dazu, dass der Einfluss des Unterdrucks auf das Kreislaufsystem ausgeglichen wird, während die positive Wirkung des Unterdrucks auf die Durchblutung des Fettgewebes erhalten bleibt.

Von Vorteil kann dabei sein, wenn die Liegeeinrichtung der Länge verschiebbar nach innerhalb des Gehäuses angeordnet ist. Auf diese Weise wird das Training in verschiedenen Positionen ermöglicht. So kann einerseits gezielt eine Einwirkung auf die Beine und das Gesäß oder andererseits auf Bauch und Hüfte realisiert werden.

Hierbei hat es sich als günstig erwiesen, wenn ein Sitz auf der Liege angeordnet ist. Der Sitz verhindert hierbei das Hineinrutschen in die Unterdruckkammer, bedingt durch die Sogkraft des Unterdrucks.

Ferner sind auf der Liege Abstützeinrichtungen für die Schultern der Person angeordnet. Der Schulterhalter verhindert hierbei das Herausrutschen der Person in der Phase des Überdrucks oder bei abgesenkter Position.

Als günstig hat es sich erwiesen, wenn die Dichtmanschette als aufblasbarer Dichtring ausgebildet ist und die Öffnung gegenüber der Liegeeinrichtung und der Person abdichtet. Ein aufblasbarer Dichtring passt sich hierbei optimal der jeweiligen Person an und dichtet die Öffnung insbesondere auch zwischen Öffnung und Liege ab.

Vorzugsweise ist die Dichtmanschette als Vakuumdecke ausgebildet. Solche Vakuumdecken gibt es in verschiedenen Größen, so dass sie einfach auf die Größe bzw. die Figur der jeweiligen Person, z. B. Bauch-, Taillen- oder Hüftdurchmesser, abgestimmt werden kann. Die Vakuumdecke wird hierbei von der Person außerhalb des Gerätes angezogen und dann auf einen Dichtungsring der Öffnung gezogen.

Besonders günstig ist es, wenn die Arbeitseinrichtung als Stepper, Zimmerfahrrad oder Ellipsentrainer ausgebildet ist. Diese Arbeitsgeräte sind gleichermaßen im Sitzen als auch im Liegen betätigbar. Hierbei dient der Sitz bei der Ausgestaltung als Stepper zur Abstützung des Körpers, damit auf die Beine kein hoher Druck ausgeübt werden muss. Gleichermaßen wird bei der Ausbildung als Ellipsentrainer, die Beine vom Anpressdruck bedingt aus Körpergewicht und Sogkraft zu entlasten.

in einer weiteren Ausführungsform ist vorgesehen, dass die Arbeitseinrichtung durch einen Elektromotor angetrieben ist und durch Krafteinwirkung der Person gebremst wird. Auf diese Weise ist auch ein passiver Betrieb möglich, bei dem eine besonders genaue Einstellung der Belastung der Person durchgeführt werden kann.

Darüber hinaus kann vorgesehen sein, dass eine Steuerungseinrichtung außerhalb des Gehäuses angeordnet ist. Auf diese Weise kann das Gerät einfach von der Person oder durch betreuende Personen bedient werden. Über ein Terminal mit Bedienungseinheiten und wenigstens einem Display, das mit der Steuereinrichtung verbunden ist, kann die trainierende Person sämtliche Werte des derzeitigen Betriebszustandes der erfindungsgemäßen Vorrichtung sowie physiologische Werte abfragen und darstellen lassen, z. B. den Druck, die Tretkadenz, die geleistete und noch zu leistende Arbeit, Herzfrequenz, Blutdruck. Außerdem kann die trainierende Person über das Terminal die Belastungszustände programmieren. Um eine gute Zugänglichkeit des Terminals zu ermöglichen, ist dieses auf einem beweglichen Arm angeordnet.

Gemäß einer weiteren Ausgestaltung hat es sich als günstig erwiesen, wenn innerhalb des Gehäuses wenigstens eine zusätzliche Druckkammer vorgesehen ist, die einen von der Hauptkammer unterschiedlichen Druck aufweisen kann. Hierbei kann die zusätzliche Kammer im Bereich des Rumpfes, insbesondere des Bauches der Person, angeordnet sein. Auf diese Weise ist es möglich, einen unterschiedlichen Druck auf verschiedene Körperteile auszuüben. Die zweite Kammer kann hierbei bereits innerhalb des Gehäuses angeordnet sein, oder der Person außerhalb des Gehäuses angelegt werden. Durch die zweite Kammer ist z. B. eine Überdruckeinwirkung auf den Bauch möglich, während die Beine einer Unterdruckeinwirkung ausgesetzt sind, oder umgekehrt.

Eine andere Ausführungsform sieht vor, dass in mindestens einer Kammer Magnetspulen vorgesehen sind, um ein Magnetfeld im Bereich der Person zu erzeugen. Die Magnetspulen können hierbei z. B. den Bauch oder die Beine der Person umschließen. Auf diese Weise können Magnetfelder zur Unterstützung der therapeutischen Wirkung herangezogen werden.

Es hat sich femer vorteilhaft erwiesen, wenn in mindestens einer Kammer Lichtquellen zur Bestrahlung der Person vorgesehen sind. Dazu können beispielsweise über Lichtleiter Strahlen unterschiedlicher Wellenlängen, einschließlich Infrarot und Ultraviolett, eingespeist werden. Die Bestrahlung kann gezielt auf vorbestimmte Körperareale gerichtet werden.

Eine wesentliche Beeinflussung kann weiterhin dadurch erreicht werden, dass eine Klimaeinrichtung zur Einstellung der Temperatur und der Luftfeuchtigkeit vorgesehen ist. Die Selektivität der Anwendung wird dadurch erhöht, dass die Klimaeinrichtung jede einzelne Kammer unabhängig voneinander klimatisieren kann.

Ferner kann eine erste Vakuumpumpe zur Evakuierung der Hauptkammer und je eine Vakuumpumpe für jede weitere Kammer vorgesehen sein. Auf diese Weise wird eine größtmögliche Unabhängigkeit bei der Steuerung des Unterdrucks erreicht.

Eine kostengünstige Variante dieser Ausführungsform kann dadurch erreicht werden, dass eine einzige Vakuumpumpe vorhanden ist und mit jeder einzelnen Kammer über ein gesondertes Druckregelungsventil verbunden ist.

Ferner stellt die Erfindung ein Verfahren zur Körperertüchtigung mittels einer Vorrichtung mit einem Gehäuse, in dem eine mit den Beinen betätigbare Arbeitseinrichtung und ein Sitz zur Abstützung des Körpers der Person angeordnet ist, einer Öffnung in dem Gehäuse, welche den Körper der Person umschließt, einer Dichtmanschette zur Abdichtung des Gehäuses im Bereich der Öffnung gegenüber dem Körper der Person, und einer Vakuumpumpe zur Erzeugung eines Unterdrucks in dem Gehäuse zur Verfügung, wobei zunächst eine Tretbewegung auf die Arbeitseinrichtung ausgeübt wird und erst zeitversetzt die Einschaltung der Vakuumpumpe auslöst wird.

Ferner kann vorgesehen sein, dass das Absenken des Druckes an den Kreislauf der Person angepasst wird. Hierzu ist in die erfindungsgemäße Vorrichtung ein Gerät integriert, das neben dem Puls auch den Blutdruck und/oder die Atemfrequenz und/oder die Herzfrequenz der trainierenden Person misst und in Signalform ausgibt. Diese Messgeräte können in einer Weiterbildung der Erfindung mit der Steuereinheit datenübertragend verbunden sein, welche die Belastung in Abhängigkeit von den gemessenen körperlichen Belastungswerten steuert. Bei einer automatischen Steuerung hat es sich besonders bewährt die Pulsfrequenz oder die Herzfrequenz zugrunde zu legen. Hierbei kann z. B. von der trainierenden Person ein Pulsgurt oder ähnliches getragen werden, welcher mit der Steuerungseinrichtung verbunden ist und aufgrund der empfangenen Daten das Trainingsprogramm bestimmt.

Es hat sich weiterhin als günstig herausgestellt, wenn der Unterdruck in vorgegebenen zeitlichen Abständen um vorbestimmte Intervalle gesenkt wird.

Weiterhin ist vorteilhaft, wenn nach Erreichen eines vorgegebenen maximalen Unterdrucks, ein ständiger Wechsel zwischen normalem Druck und Unterdruck über vorbestimmte zeitliche Intervalle erzeugt wird.

Von Vorteil ist hierbei, wenn der Tretwiderstand und/oder der Unterdruck in Abhängigkeit von der Pulsfrequenz der Person gesteuert werden.

Ferner können der Tretwiderstand und die Intervallschaltung miteinander gekoppelt werden.

Aus dem Vorhergehenden wird deutlich, dass das Verfahren aus einer Abstimmung von Tretwiderstand, Tretbewegung und Unterdruckwirkung immer unter Rücksichtnahme auf die Pulsfrequenz der Person besteht.

Zu Beginn des Verfahren findet ausschließlich eine Tretbewegung statt, in dem Gehäuse herrscht normaler Druck. Erst nach dem Beginn der Tretbewegung wird die Pumpe zugeschaltet und ein Unterdruck erzeugt. Auf diese Weise wird verhindert, dass eine Unterdruckeinwirkung ohne Tretbewegung stattfindet, da sonst das Blut durch den Unterdruck in die Beine gezogen wird aber durch die Muskeln zuwenig zurücktransportiert wird, was zu einer Unterversorgung des Gehirns und zur Bewusstlosigkeit führen könnte. Hierzu ist eine Überwachungseinrichtung vorgesehen, welche die Tretbewegung überwacht und die Stromzufuhr an die Vakuumpumpe unterbricht oder ein oder mehrere Ventile im Unterdruckbereich öffnet, wenn über eine vorbestimmte Zeit keine Tretbewegung mehr stattfindet.

Vorzugsweise wird der Tretwiderstand auf die jeweilige Person abgestimmt und speziell eingestellt. Beispielsweise wird der Tretwiderstand bei der Behandlung von Problemstellen im Bereich der Oberschenkel auf 0,8 bis maximal 1,2 Watt pro Kilogramm Körpergewicht eingestellt. In diesem Fall wird die Sitzposition in dem Gehäuse auf die hohe Position eingestellt. Bei Behandlung von Problemstellen im Bereich der Hüfte wird der Tretwiderstand auf mindestens 1 bis maximal 2 Watt pro Kilogramm Körpergewicht eingestellt. Hierbei nimmt die Person eine tiefe Sitzposition ein, damit auch Bauch und Hüfte der Unterdruckwirkung ausgesetzt sind.

Um eine optimale Wirkung zu erzielen, ist eine gleichmäßige Tretbewegung Voraussetzung. Als besonders geeignet haben sich hierbei 50 bis 70 Umdrehungen pro Minute erwiesen.

Wie bereits beschrieben, werden die ersten Minuten ohne Unterdruckwirkung getreten, um den Kreislauf anzuregen. Diese erste Phase dauert ca. 3 Minuten. Anschließend wird der atmosphärische Druck im Gerät auf vorerst 10 Millibar abgesenkt und alle 4 bis 5 Minuten um weitere 5 bis 10 Millibar. Das Absenken des Druckes , wie auch die Zeitintervalle werden hierbei an den Kreislauf der Person angepasst. Bei kreislaufstabilen Personen wird der Unterdruck auf 30 bis maximal 50 Millibar angehoben, bei kreislaufschwachen Personen beträgt der Bereich 20 bis 25 Millibar.

Ist ein Unterdruck von 20 Millibar erreicht, beginnt die Intervallschaltung. Bei dieser Intervallschaltung findet ein ständiger Wechsel zwischen abgesenktem Druck und normalem Druck im Gerät statt. Gleichermaßen könnte hier jedoch auch anstelle eines normalen Drucks ein erhöhter Druck ausgeübt werden.

Die Intervalle werden hierbei jeweils auf die Person abgestellt und liegen im allgemeinen zwischen 10 und 90 Sekunden absaugen, gefolgt von 10 bis 30 Sekunden Belüftung, bzw. 10 bis 90 Sekunden Überdruck.

Die Intervallschaltung kann hierbei mit dem Tretwiderstand gekoppelt werden. Während der Unterdruck auf die Person einwirkt, wird der Tretwiderstand erhöht, wird das Gehäuse belüftet, verringert sich der Tretwiderstand. Aber auch die umgekehrte Koppelung kann je nach Bedarf der Person gewählt werden.

Da der Unterdruck eine Kreislaufbelastung darstellt, müssen bei zu hohem Puls sowohl der Tretwiderstand als auch der Unterdruck verringert werden.

Bevorzugte Ausführungsformen der Erfindung werden nachfolgend unter Bezugnahme auf die beigefügte Zeichnung näher erläutert. Es zeigt:
- Fig. 1: schematisch eine axonometrische Ansicht einer Vorrichtung zur sitzenden Benutzung,
- Fig. 2: die in Fig. 1 dargestellte Vorrichtung teilweise im Schnitt,
- Fig. 3: die in Fig. 1 dargestellte Vorrichtung mit einer zusätzlichen Einstieghilfe,
- Fig. 4: eine Skizze zur Bedienung der in Fig. 1- 3 dargestellten Vorrichtung,
- Fig. 5 und 6: zwei alternative Schaltdiagramme zur Ausführung der Erfindung,
- Fig. 7: eine schematische Darstellung einer erfindungsgemäßen Vorrichtung zur liegenden Benutzung im Schnitt,
- Fig. 8: die in Fig. 7 dargestellte Vorrichtung im zugeklappten Zustand,
- Fig. 9: die in Fig. 7 dargestellte Vorrichtung, wobei ein Person in der Vorrichtung liegt.
- Fig. 10: die in Fig. 9 dargestellte Vorrichtung im zugeklappten Zustand,
- Fig. 11: die in den Fig. 7 bis 10 dargestellte Vorrichtung im geneigten Zustand; und
- Fig. 12: die in den Fig. 7 bis 11 gezeigte Vorrichtung in einer Explosionsdarstellung.

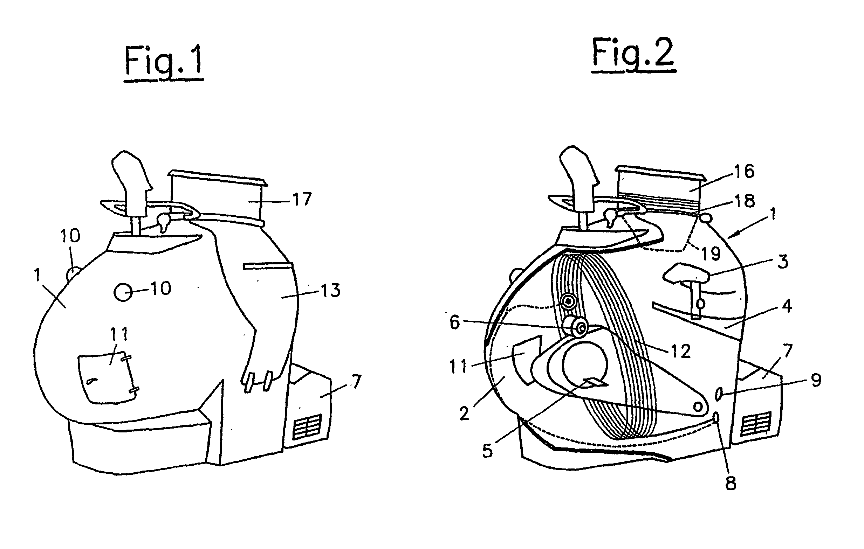

In Fig. 1 ist eine Trainingsvorrichtung dargestellt, die für sitzende Anwendungen geeignet ist.

Die Vorrichtung umfasst hierbei ein Gehäuse 1, das eine Hauptkammer 2 umschließt, in der ein Sitz 3 auf einer Verstellschiene angeordnet ist. Ferner ist in der Hauptkammer 2 eine Arbeitseinrichtung in der Form eines Zimmerfahrrades 5 angeordnet, das über einen Elektromotor 6 antreibbar ist. Luftdicht verschließbare Bedienungsöffnungen 11 ermöglichen es beispielsweise, die Beine einer Person, die bewegungsbehindert ist, von außen an den Pedalen des Zimmerfahrrades 5 zu befestigen.

An die Kammer 2 ist ein Kasten 7 angeschlossen, der eine im Detail nicht dargestellte Vakuumpumpe und eine Steuerungseinrichtung zur Ansteuerung der zusätzlichen Funktionen, die nachfolgend beschrieben werden, enthält.

Über eine Öffnung 8 kann aus der Hauptkammer 2 Luft abgesaugt werden, um einen Unterdruck zu erzeugen. Eine weitere Öffnung 9 dient zur Klimatisierung, d.h. insbesondere zur Kühlung der Hauptkammer 2 und zur Einstellung der Luftfeuchtigkeit.

Lichtquellen 10 dienen zur Bestrahlung der Beine der die Vorrichtung benutzenden Person. Magnetspulen 12 sind dazu ausgebildet, ein therapeutisch vorteilhaftes Magnetfeld im Bereich der Beine der Person aufzubauen.

Eine Einstiegstür 13, die sich nach unten hin öffnet, ermöglicht einen leichten Zugang zu der Vorrichtung. Hierbei ist die Einstiegstür 13 im Bereich der Öffnung 8 angeordnet. Im ausgeklappten Zustand kann vorgesehen sein, dass die Einstiegstür in dem im ausgeklappten Zustand sichtbaren Bereich über Erhebungen verfügt, die von der die Vorrichtung benutzenden Person als Treppe verwendet werden können und so ein bequemeres Einsteigen ermöglichen.

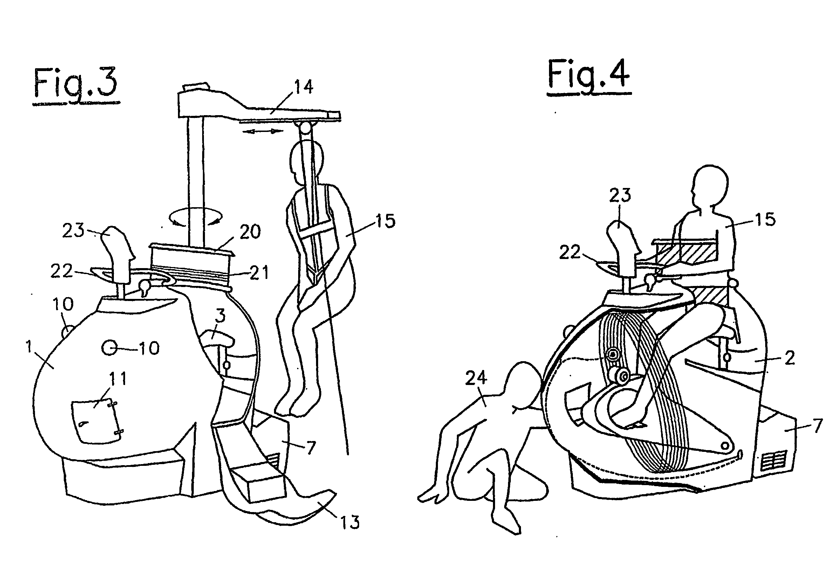

Wie insbesondere aus Fig. 3 ersichtlich, ist es möglich, gehbehinderte Personen mit einer Einstieghilfe in Form eines Kranes 14 in die Vorrichtung hineinzuheben. Die Person ist hierbei mit Bezugszeichen 15 angedeutet.

Oberhalb der Hauptkammer 2 ist eine weitere Kammer 16 angeordnet, die durch einen zylindrischen Fortsatz 17 gebildet wird. Im Bereich des Übergangs des. Gehäuses 1 zum Fortsatz 17 ist eine Öffnung 18 ausgebildet, die eine Dichtmanschette 19 aufweist, durch welche die Kammer 2 gegenüber der Person 15 im Hüftbereich abgedichtet wird. Die Dichtmanschette 19 kann in der Form eines Höschens mit zwei Öffnungen für die Beine der Person ausgebildet sein.

Eine weitere Dichtmanschette 20 dient zur Abdichtung der weiteren Kammer 16 gegenüber dem Körper im oberen Bereich des Brustkorbs. Eine weitere Magnetspule 21 ist vorgesehen, um im Brustbereich ein Magnetfeld zu erzeugen. Die weitere Kammer 16 ist nicht nur getrennt von der Hauptkammer 2 mit unter Druck beaufschlagbar, sondern auch getrennt klimatisierbar und besitzt eigene Lichtquellen.

Griffe 22 dienen zur Abstützung der Person und zur Aufnahme einer Anzeigeeinheit 23.

In der Fig. 4 ist eine Person 24 dargestellt, die durch die Bedienungsöffnungen 11 die Beine der Person 15 an der Arbeitseinrichtung 5 befestigt. Insbesondere bei gehbehinderten Personen kann es sich als besonders vorteilhaft erweisen, wenn die Arbeiteinrichtung hierbei mit Fußbefestigungen versehen ist, so dass die Füße an der Arbeitseinrichtung gehalten werden.

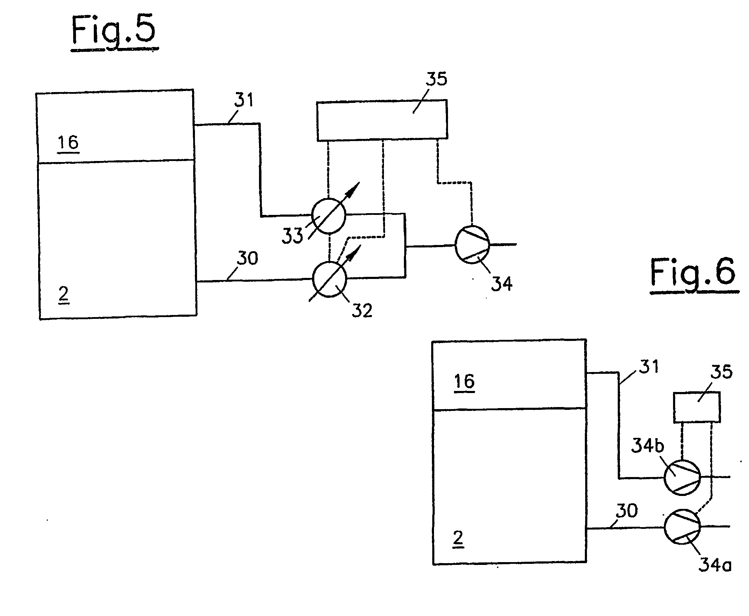

Wie in Fig. 5 dargestellt, führt aus der Hauptkammer 2 eine erste Leitung 20 und aus der weiteren Kammer 16 eine zweite Leitung 21 zu einem ersten bzw. zweiten Druckregelungsventil 22, 23. An der stromabwärtigen Seite der Druckregelungsventile 22, 23 ist eine Vakuumpumpe 24 angeordnet, um die Kammern 2 und 16 zu evakuieren. Eine Steuerungseinrichtung 25 steuert die Druckregelungsventile 22, 23 und die Vakuumpumpe 24. Auf diese Weise kann in den Kammern 2 und 16 der jeweils gewünschte Druck eingestellt werden.

Die in Fig. 6 dargestellte Ausführungsform unterscheidet sich von der Fig. 5 dadurch, dass für jede Kammer 2 und 16 eine eigene Vakuumpumpe 24a und 24b vorgesehen ist, die von der Steuerungseinrichtung 25 getrennt ansteuerbar sind.

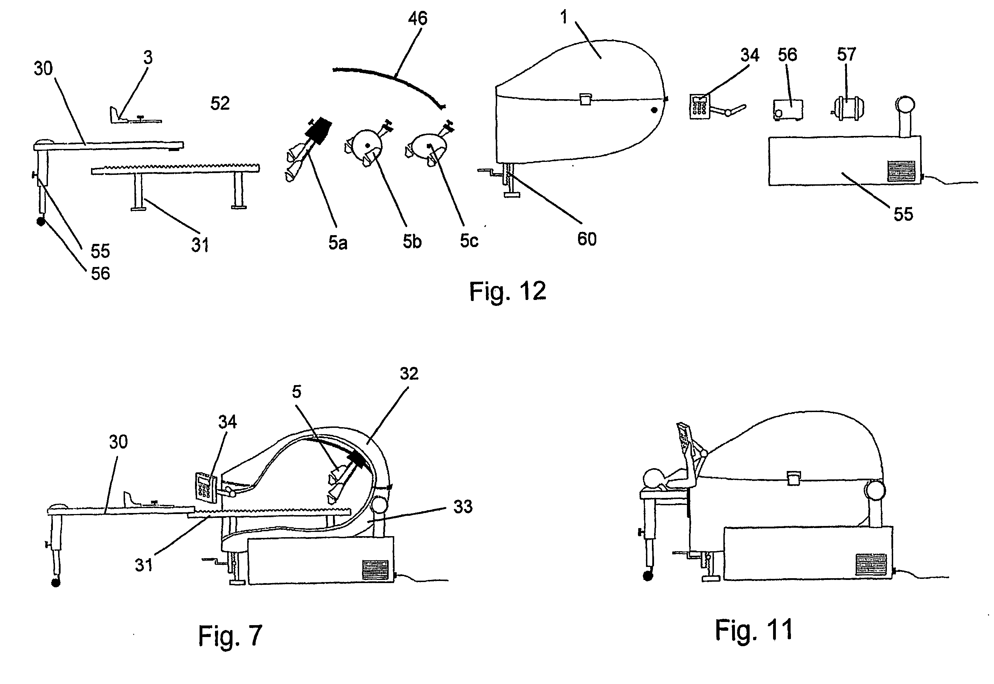

Eine Vorrichtung gemäß der Erfindung ist in den Fig. 7 bis 11 dargestellt. Bei der in diesen Figuren dargestellten Vorrichtung handelt es sich um ein Trainingsgerät, das von der jeweiligen Person im Liegen zu benutzen ist.

Bei der Beschreibung der Zeichnungen werden hierbei gleiche Bauelemente mit gleichen Bezugszeichen wie in den Fig. 1 bis 6 versehen.

Die in Fig. 7 dargestellte Vorrichtung besteht aus einem Gehäuse 1, das eine Hauptkammer 2 umschließt. Innerhalb dieser Hauptkammer 2 ist eine Arbeitseinrichtung in Form eines Zimmerfahrrades 5 angeordnet. Das Gehäuse 1 ist hierbei solchermaßen angeordnet, dass sich die Öffnung 8 seitlich an dem Gehäuse befindet, so dass die Person, welche die Vorrichtung benutzt im Liegen von der Öffnung umfangen wird. Durch die Öffnung 8 erstreckt sich eine Liegeeinrichtung 30 bis in das Gehäuse. Über ein Gestell 31 mit einem Rastelement, welches innerhalb des Gehäuses angeordnet ist, sich bis über die Öffnung 8 aus dem Gehäuse 1 hinaus erstreckt und welches mit der Unterseite der Liegeeinrichtung 30 im Eingriff steht, kann die Liegeeinrichtung 30 in verschiedene Positionen innerhalb des Gehäuses eingefahren werden. Diese Einstellung ermöglicht einmal eine Einwirkung ausschließlich auf die Beine und das Gesäß und ein anderes mal eine Einwirkung auf Bauch und Hüfte.

Auf der Liege 30 ist ein Sitz 3 angeordnet, der als Rastelement ausgebildet ist, und der sich bei der Benutzung zwischen den Beinen der Person befindet. Um hier auf die verschiedenen Körpergrößen der zu trainierenden Personen Rücksicht zu nehmen, ist dieser Sitz vorzugsweise auf der Liege verschieblich angeordnet. Der Sitz auf der Liege verhindert hierbei das Hineinziehen der Person in das Gehäuse in der Phase des Unterdrucks.

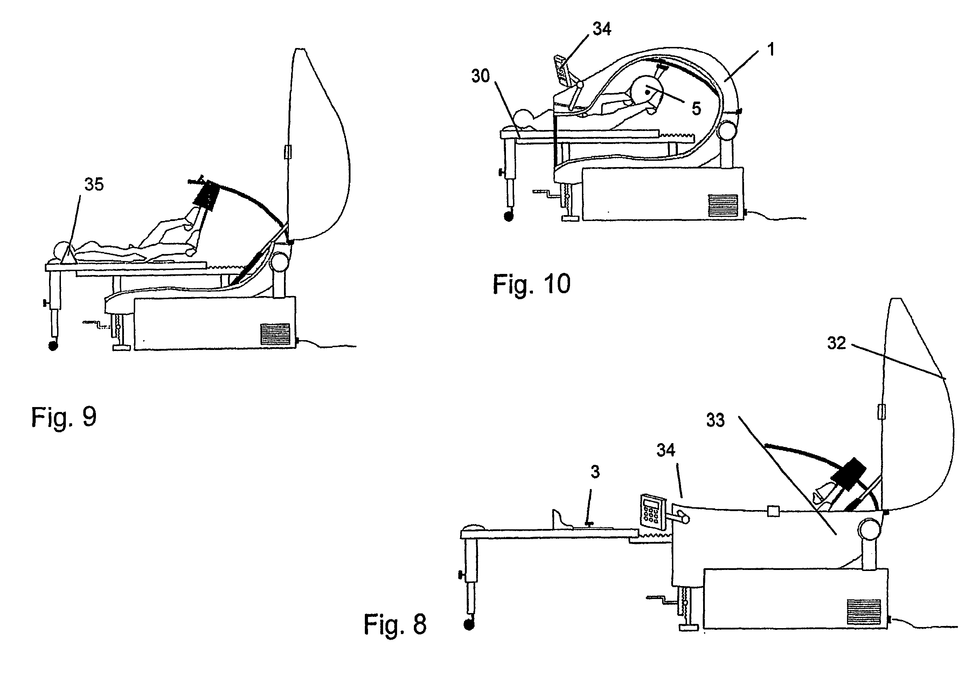

Um ein leichteres Einsteigen zu ermöglichen, kann die Vorrichtung, wie in Fig. 8 gezeigt, einfach in zwei Halbschalenelemente aufgeklappt werden, wobei die beiden Halbschalenelemente 32 und 33 im Bereich der Öffnung 8 voneinander getrennt werden.

In Fig. 9 ist die Vorrichtung in Benutzung dargestellt. Hierbei hat sich bereits eine Person auf die Liege 30 gelegt, und die Liege 30 befindet sich an der gewünschte Position innerhalb des Gehäuses. Die Person wird hierbei, wie bereits erwähnt, einerseits durch den Sitz 3 abgestützt. Die Person wird ferner durch Abstützungen 35 auf der Liege 30 gesichert, wobei die Abstützungen oberhalb der Schulter der zu behandelnden Person angeordnet werden. Diese Abstützungen sind insbesondere dann notwendig, wenn die Liege aus der waagerechten Position in eine andere Position geneigt wird, wie z. B. in Fig. 11 dargestellt ist. Außerdem bewirkt die Schulterstütze, dass im Betrieb mit überdruck die Person nicht aus der Vorrichtung gedrückt wird. Im Betrieb kann die Liege in beliebig geneigte Positionen bis in die senkrechte Position gebracht werden.

Außerhalb des Gehäuses ist eine Steuerungseinheit 34 angeordnet, die entweder von der trainierenden Person selbst bedient werden kann oder von einer betreuenden Person. Zu diesem Zweck ist die Steuerungseinheit 34 mit einem armähnlichen Klappmechanismus versehen um diesen in verschiedene Positionen zu bewegen.

Zur Abdichtung der Person gegen die Öffnung sowie gegen die Liege kann vorgesehen sein, dass die Person außerhalb des Gerätes einen aufblasbaren Innenring anlegt, der in ausgelassenem Zustand angelegt wird, und erst nach Einnehmen der Endposition auf der Liege aufgeblasen wird. Gleichermaßen ist es möglich, dass die Person eine Vakuumdecke anlegt, welche die Rolle der Abdichtung übernimmt.

Es hat sich hierbei besonders bewährt, wenn verschiedene Abdichtungen zusammenwirken um eine vollständige Abdichtung der Liege 30 gegen die Öffnung 8 und der Person 15 gegen die Liege 30 und die Öffnung 8 zu realisieren. Hierbei kann der Bereich zwischen der Unterseite der Liegeeinrichtung 30 und dem diese umgebenden Bereiche der Öffnung 8 von einer separaten Dichtungseinrichtung abgedichtet werden.

Aus Fig. 12 geht der modulare Aufbau der Vorrichtung der Fig. 7 bis 11 hervor. Der modulare Aufbau ermöglicht eine schnelle und flexible Anpassung an verschiedene Trainingssituationen und führt zu einer guten Transportmöglichkeit sowie zu einer großen Wartungsfreundlichkeit.

Die Trainingsvorrichtung umfasst als erstes Modul die verstellbare Liegevorrichtung umfassend die Liege bzw. Auflage 30 und das Gestell 31 sowie die Schulterstütze 35 und dem Sitz 3. Sitz und Schulterstütze sind längsverschieblich auf der Liege 30 angeordnet.

Das Gestell 31 ist dabei im wesentlichen ortsfest, während die Liege 30, auf welcher der Körper während des Trainings ruht, gegenüber dem Gestell 31 beweglich und, wie oben bereits ausgeführt wurde, höhen-, längen- und neigungsverstellbar und zu Reinigungszwecken leicht entfernbar ist. Zur Verstellung weist die Liege verstellbare Füße 50 mit Rollen 51 auf. Das Gestell ist auf seiner Oberseite mit Rasten 52 versehen, die in entsprechende Gegenrasten der Liege 30 eingreifen und diese in einer bestimmten Längsverstellung arretieren.

Als zweites Modul sind untereinander austauschbare Arbeitsvorrichtungen 5a, 5b, 5c vorgesehen, die allesamt im Liegen betrieben werden können. Gezeigt sind in der Fig. 12 ein Stepper 5a, ein Fahrradtrainer 5b und ein Ellipsoidtrainer 5c. Die Arbeitsvorrichtungen 5a-c sind an einer Einstellvorrichtung in Form einer Schiene 46 verschieblich gehalten. Die Arbeitsvorrichtungen 5a-c weisen allesamt Daten- oder Signalübertragungseinrichtungen auf, die mit einer Steuereinrichtung verbunden werden können. Außerdem können die Arbeitsvorrichtungen 5a-c optional mit einem Elektromotor versehen sein.

Die Verstelleinrichtung 46 ermöglicht eine korrekte ergonometrische Einstellung der Arbeitsvorrichtung unabhängig von der Stellung der Liege. Somit ist sowohl bei weitgehend in das Gehäuse 1 eingefahrener Liege 30 (ein großer Bereich des Körpers ist dem Druck ausgesetzt) als auch bei weitgehend aus dem Gehäuse gefahrener Liege 30 (ein kleiner Bereich des Körpers ist dem Druck ausgesetzt), stets eine gelenkschonende Positionierung der Person zur Arbeitsvorrichtung möglich.

Ein weiteres Modul stellt die Steuereinrichtung bzw. das Terminal 34 dar.

Die Versorgungseinheit 55 stellt ein weiteres Modul dar. In der Versorgungseinheit sind die Vorrichtungen für die Klimakontrolle, beispielsweise eine Klimaanlage 56, sowie eine Pumpe 57 zur Erzeugung von Unter- bzw. Überdruck in der Kammer 2 untergebracht. Die Versorgungseinheit 55 besteht aus einem Gehäuse 58, das eine Schallkapselung aufweist, so dass für den Betrieb in Rehabilitationszentren oder Fitnessanlagen möglichst wenig Geräusche im Betrieb erzeugt werden.

Das letzte Modul wird vom Gehäuse gebildet, das wie oben bereits beschrieben aufgebaut ist. Das Gehäuse 1 ruht auf einem höhenverstellbaren Gestell 60, das am Boden verankert ist.

Aufgrund des modularen Aufbaus kann die Vorrichtung der Fig. 7-11 zum Training in Liegen mit einem Austausch von nur einer geringen Anzahl von Teilen in eine Vorrichtung der Fig. 1-4 zum Trainieren im Sitzen umgebaut werden.

In einer diesbezüglich besonders vorteilhaften Ausgestaltung ist das Gehäuse dabei so ausgebildet, dass es sowohl zum Trainieren im Liegen als auch zum Trainieren im Sitzen verwendet werden kann. Hierzu muss die Öffnung lediglich nach oben gedreht werden, nachdem die Liege herausgenommen und durch einen Sitz ersetzt wurde.

Die in den Figuren 7 bis 12 dargestellte Vorrichtung kann gleichermaßen wie die in Bezug auf die Fig. 1 bis 6 beschriebene Vorrichtung mit zusätzlichen integrierten Magnetspulen, mit Klimaeinrichtungen oder Lichtbestrahlung ausgestattet sein.

In der Vorrichtung lassen sich sowohl Über- als auch Unterdruck gleichmäßig oder stufenförmig über einen bestimmten Zeitraum steigern bzw. absenken. In verschiedenen Behandlungsphasen schaltet eine Intervallschaltung den Über- bzw. Unterdruck in unterschiedlichen Zeiträumen ein bzw. aus. Über- und Unterdruck können in einer Behandlungsphase abwechseln. Ober- und Unterdruck können hierbei sowohl manuell als auch automatisch gesteuert werden. Bezüglich der automatischen Steuerung kann vorgesehen sein, dass diese Steuerung aufgrund der Herzfrequenz oder des Blutdrucks der behandelnden Person oder aufgrund des Temperaturverhaltens in der Unterdruckkammer oder an der Oberfläche der Haut der trainierenden Person, oder dem Widerstand des Ergometers berechnet wird.

Die vorliegende Erfindung ermöglicht es, ein gezielteres Training und eine den Eigenschaften der jeweiligen Person besser angepasste Therapie zu verwirklichen.

## Patentansprüche

1. Vorrichtung zum Schlankmachen von Personen durch Verbesserung der Hautdurchblutung mittels Unterdruck ohne zusätzliche Gelenkbelastung mit
- einem Gehäuse (1), welches eine Druckkammer (2) bildet, in der eine mit den Beinen betätigbare Arbeitseinrichtung (5) angeordnet ist,
- einer Öffnung (8) in dem Gehäuse (1), welche den Körper der Person umschließt,
- einer Dichtmanschette (19) zur Abdichtung des Gehäuses (1) im Bereich der Öffnung (8) gegenüber dem Körper der Person,
- einer Vakuumpumpe zur Erzeugung eines Unterdrucks in dem Gehäuse (1), **dadurch gekennzeichnet, dass** eine Liegeeinrichtung (30) vorgesehen ist, durch die der Körper der Person abstützbar ist, wobei die Person während der Betätigung der Arbeitseinrichtung eine liegende Position auf der Liegeeinrichtung einnimmt.

2. Vorrichtung nach Anspruch 1, **dadurch gekennzeichnet, dass** sich die Liegeeinrichtung (30) durch eine im Wesentlichen seitlich angeordnete Öffnung (9) in das Gehäuse erstreckt.

3. Vorrichtung nach Anspruch 1 oder 2, **dadurch gekennzeichnet, dass** die Liegeeinrichtung (30) der Länge nach verschiebbar innerhalb des Gehäuses (1) angeordnet ist.

4. Vorrichtung nach einem der oben genannten Ansprüche, **dadurch gekennzeichnet, dass** die Liegeeinrichtung (30) eine Verstelleinrichtung zur Neigung der Liegeeinrichtung aufweist.

5. Vorrichtung nach einem der oben genannten Ansprüche, **dadurch gekennzeichnet, dass** ein Sitz (3) auf der Liegeeinrichtung (30) angeordnet ist.

6. Vorrichtung nach einem der oben genannten Ansprüche, **dadurch gekennzeichnet, dass** auf der Liegeeinrichtung (30) Abstützeinrichtungen (35) für die Schultern der Person angeordnet sind.

7. Vorrichtung nach einem der oben genannten Ansprüche, **dadurch gekennzeichnet, dass** die Dichtmanschette (19) als aufblasbarer Dichtring ausgebildet ist und die Öffnung (8) gegenüber der Liegeeinrichtung (30) und der Person abdichtet.

8. Vorrichtung nach einem der oben genannten Ansprüche, **dadurch gekennzeichnet, dass** die Dichtmanschette (19) als Vakuumdecke ausgebildet ist.

9. Vorrichtung nach einem der oben genannten Ansprüche, **dadurch gekennzeichnet, dass** das Gehäuse (1) als Rundkörper, insbesondere eiförmig, ausgebildet ist.

10. Vorrichtung nach Anspruch 9, **dadurch gekennzeichnet, dass** das Gehäuse (1) in Halbschalen teilbar ist, wobei jede Halbschale einen Teil der Öffnung (8) aufweist, die durch ein Verschlusssystem miteinander verbunden werden.

11. Vorrichtung nach einem der oben genannten Ansprüche, **dadurch gekennzeichnet, dass** die Arbeitseinrichtung (5) als Stepper, Zimmerfahrrad oder Ellipsentrainer ausgebildet ist.

12. Vorrichtung nach einem der oben genannten Ansprüche, **dadurch gekennzeichnet, dass** eine Steuerungseinrichtung (34) außerhalb des Gehäuses angeordnet ist.

13. Vorrichtung nach einem der oben genannten Ansprüche, **dadurch gekennzeichnet, dass** innerhalb des Gehäuses wenigstens eine Hauptkämmer (2) und wenigstens eine zusätzliche Druckkammer vorgesehen ist, die von der Hauptkammer im wesentlichen druckdicht abgetrennt und mit einem von der Hauptkammer (2) unterschiedlichen Druck beaufschlagbar ist.

14. Vorrichtung nach Anspruch 13, **dadurch gekennzeichnet, dass** die zusätzliche Druckkammer im Bereich des Rumpfes, insbesondere des Bauches der Person, angeordnet ist.

15. Vorrichtung nach einem der oben genannten Ansprüche, **dadurch gekennzeichnet, dass** in mindestens einer Kammer Magnetspulen (12) vorgesehen sind, um ein Magnetfeld im Bereich der Person zu erzeugen.

16. Vorrichtung nach Anspruch 15, **dadurch gekennzeichnet, dass** die Magnetspulen (12) den Bauch der Person umschließen.

17. Vorrichtung nach Anspruch 15, **dadurch gekennzeichnet, dass** die Magnetspulen (12) die Beine der Person umschließen.

18. Vorrichtung nach einem der oben genannten Ansprüche, **dadurch gekennzeichnet, dass** in mindestens einer Kammer lichtquellen (10) zur Bestrahlung der Person vorgesehen sind.

19. Vorrichtung nach einem der oben genannten Ansprüche, **dadurch gekennzeichnet, dass** eine Klimaeinrichtung zur Einstellung der Temperatur und der Luftfeuchtigkeit vorgesehen ist.

## Claims

1. An apparatus for slimming persons by improving the blood flow in the skin by means of vacuum without additionally straining the joints, comprising
- a housing (1) forming a pressure chamber (2), in which a working means (5) operable by the legs is arranged,
- an opening (8) in the housing (1), which surrounds the person's body,
- a sealing collar (19) for sealing the housing (1) in the area of the opening (8) against the person's body,
- a vacuum pump for generating a vacuum in the housing (1),
**characterized in that** a lounger means (30) is provided by which the body can be supported, wherein the person assumes a lying position on the lounger means when operating the working means.

2. An apparatus as claimed in claim 1, **characterized in that** the lounger means (30) extends through a substantially laterally arranged opening (9) into the housing.

3. An apparatus as claimed in claim 1 or 2, **characterized in that** the lounger means (30) is arranged longitudinally displaceable within the housing (1).

4. An apparatus as claimed in one of the preceding claims, **characterized in that** the lounger means (30) comprises an adjustment means for inclining the lounger means.

5. An apparatus as claimed in one of the preceding claims, **characterized in that** a seat (3) is arranged on the lounger means (30).

6. An apparatus as claimed in one of the preceding claims, **characterized in that** supporting means (35) for the person's shoulders are arranged on the lounger means (30).

7. An apparatus as claimed in one of the preceding claims, **characterized in that** the sealing collar (19) is formed as an inflatable sealing ring and seals the opening (8) against the lounger means (30) and the person.

8. An apparatus as claimed in one of the preceding claims, **characterized in that** the sealing collar (19) is formed as a vacuum cover.

9. An apparatus as claimed in one of the preceding claims, **characterized in that** the housing (1) is formed as a round body, in particular in the shape of an egg.

10. An apparatus as claimed in claim 9, **characterized in that** the housing (1) can be divided into half-shells, wherein each half-shell comprises a portion of the opening (8), which are joined by a closure system.

11. An apparatus as claimed in one of the preceding claims, **characterized in that** the working means (5) is formed as a stepper, a room bicycle or an ellipse trainer.

12. An apparatus as claimed in one of the preceding claims, **characterized in that** a control means (34) is arranged outside of the housing.

13. An apparatus as claimed in one of the preceding claims, **characterized in that** within the housing at least one main chamber (2) and at least one additional pressure chamber are provided, which is substantially pressure-tight sealed against the main chamber and which can be loaded by a pressure that is different to the pressure in the main chamber (2).

14. An apparatus as claimed in claim 13, **characterized in that** the additional pressure chamber is arranged in the area of the trunk, in particular in the area of the abdomen of the person.

15. An apparatus as claimed in one of the preceding claims, **characterized in that** magnetic coils (12) are provided at least in one chamber in order to generate a magnetic field in the area of the person.

16. An apparatus as claimed in claim 15, **characterized in that** the magnetic coils (12) encompass the abdomen of the person.

17. An apparatus as claimed in claim 15, **characterized in that** the magnetic coils (12) encompass the legs of the person.

18. An apparatus as claimed in one of the preceding claims, **characterized in that** light sources (10) for irradiating the person are provided at least in one chamber.

19. An apparatus as claimed in one of the preceding claims, **characterized in that** an air conditioning means for adjusting the temperature and the air moisture are provided.

## Revendications

1. Dispositif d'amincissement de personnes par amélioration de l'irrigation sanguine cutanée par dépression, sans contrainte supplémentaire des articulations, comprenant
- un carter (1) qui forme une chambre de pression (2), dans laquelle est disposé un agencement de travail (5) actionnable par les jambes,
- une ouverture (8) dans le carter (1) qui entoure le corps de la personne,
- une garniture d'étanchéité (19) pour l'étanchéité du carter (1) par rapport au corps de la personne dans la zone de l'ouverture (8),
- une pompe à vide pour produire une dépression dans le carter (1),
**caractérisé en ce qu'**il est prévu un agencement de repos (30) qui permet de supporter le corps de la personne, la personne occupant une position couchée sur l'agencement de repos pendant l'actionnement de l'agencement de travail.

2. Dispositif suivant la revendication 1, **caractérisé en ce que** l'agencement de repos (30) s'étend dans le carter au travers d'une ouverture (8) disposée sensiblement latéralement.

3. Dispositif suivant l'une des revendications 1 ou 2, **caractérisé en ce que** l'agencement de repos (30) est disposé à l'intérieur du carter (1) avec une possibilité de déplacement en longueur.

4. Dispositif suivant l'une des revendications précitées, **caractérisé en ce que** l'agencement de repos (30) présente un agencement de réglage pour l'inclinaison de l'agencement de repos.

5. Dispositif suivant l'une des revendications précitées, **caractérisé en ce qu'**un siège (3) est disposé sur l'agencement de repos (30).

6. Dispositif suivant l'une des revendications précitées, **caractérisé en ce que** des agencements d'appui (35) pour les épaules de la personne sont disposés sur l'agencement de repos (30).

7. Dispositif suivant l'une des revendications précitées, **caractérisé en ce que** la garniture d'étanchéité (19) est configurée sous forme d'anneau d'étanchéité gonflable et étanchéifie l'ouverture (8) par rapport au agencement de repos (30) et à la personne.

8. Dispositif suivant l'une des revendications précitées, **caractérisé en ce que** la garniture d'étanchéité (19) est réalisée sous forme de couverture à vide.

9. Dispositif suivant l'une des revendications précitées, **caractérisé en ce que** le carter (1) est configuré sous forme de corps rond, en particulier ovoïde.

10. Dispositif suivant la revendication 9, **caractérisé en ce que** le carter (1) est séparable en demi-coquilles, chaque demi-coquille présentant une partie de l'ouverture (8), qui sont assemblées entre elles par un système de fermeture.

11. Dispositif suivant l'une des revendications précitées, **caractérisé en ce que** l'agencement de travail (5) est réalisé sous forme de marche d'appartement, de vélo d'appartement ou de vélo elliptique.

12. Dispositif suivant l'une des revendications précitées, **caractérisé en ce qu'**un agencement de commande (34) est disposé à l'extérieur du carter.

13. Dispositif suivant l'une des revendications précitées, **caractérisé en ce qu'**à l'intérieur du carter sont prévues au moins une chambre principale (2) et au moins une chambre de pression supplémentaire, qui est séparée de façon sensiblement étanche à la pression de la chambre principale et peut être alimentée en une pression différente de la chambre principale (2).

14. Dispositif suivant la revendication 13, **caractérisé en ce que** la chambre de pression supplémentaire est disposée dans la région du tronc, en particulier du ventre de la personne.

15. Dispositif suivant l'une des revendications précitées, **caractérisé en ce que** des bobines d'aimant (12) sont prévues dans au moins une chambre pour produire un champ magnétique dans la zone de la personne.

16. Dispositif suivant la revendication 15, **caractérisé en ce que** les bobines d'aimant (12) entourent le ventre de la personne.

17. Dispositif suivant la revendication 15, **caractérisé en ce que** les bobines d'aimant (12) entourent les jambes de la personne.

18. Dispositif suivant l'une des revendications précitées, **caractérisé en ce que** des sources lumineuses (10) sont prévues dans au moins une chambre pour irradier la personne.

19. Dispositif suivant l'une des revendications précitées, **caractérisé en ce qu'**un agencement de climatisation est prévu pour régler la température et l'humidité de l'air.
